# EUROPÄISCHE PATENTSCHRIFT

(11) **EP 0 963 217 B1**
(45) Veröffentlichungstag und Bekanntmachung des Hinweises auf die Patenterteilung: **15.12.2004**
(21) Anmeldenummer: 98905412.7
(22) Anmeldetag: 06.02.1998
(51) Int. Cl.: A61N 1/05

(54) **TAMPON, INSBESONDERE ZUR ANWENDUNG BEI HARN-INKONTINENZ VON FRAUEN**
TAMPON, IN PARTICULAR FOR WOMEN SUFFERING FROM URINARY INCONTINENCE
TAMPON, A UTILISER NOTAMMENT PAR DES FEMMES SOUFFRANT D'INCONTINENCE URINAIRE

(30) Priorität: 07.02.1997 DE 19704720; 23.04.1997 DE 19717086
(43) Veröffentlichungstag der Anmeldung: 15.12.1999
(73) Patentinhaber: Biobedded Systems GmbH, 46045 Oberhausen (DE)
(72) Erfinder: HILBURG, Andreas, D-46045 Oberhausen (DE); KOTOWSKI, Herbert, D-46514 Schermbeck (DE)
(74) Vertreter: Freischem, Stephan, Dipl.-Ing.
(86) Internationale Anmeldenummer: PCT/EP1998/000672
(87) Internationale Veröffentlichungsnummer: WO 1998/034677

(56) Entgegenhaltungen:
- EP-A- 0 411 632
- EP-A- 0 733 381
- WO-A-88/10106
- WO-A-93/24176
- FR-A- 2 547 203

## Beschreibung

Die Erfindung betrifft einen neuartigen Tampon, insbesondere zur Anwendung bei Harn-Inkontinenz von Frauen, bestehend aus einem hautverträglichen, elastischen Material, welches in angefeuchtetem Zustand entgegen einer Rückstellkraft komprimierbar ist, insbesondere einem Kunststoffmaterial wie Polyvinylalkohol (PVA). Bei der bevorzugten Anwendung im Falle der Harn-Inkontinenz von Frauen übt die Rückstellkraft des vaginal eingeführten Tampons einen Druck auf die Urethra aus.

Die beschriebenen elastischen Tampons werden seit langer Zeit zur Vorsorge gegen unwillkürlichen Harnverlust aufgrund einer erschlafften Bekenbodenmuskulatur eingesetzt. Der in die Vagina eingeschobene Tampon unterstützt durch seine elastischen Eigenschaften die Beckenbodenmuskulatur, so daß die Urethra zuverlässig verschlossen bleibt. Derartige Tampons sind beispielsweise aus der US-PS 4,019,498 oder der EP-PS 363 421 bekannt. Besonders geeignet für die Herstellung eines derartigen Tampons ist ein erhärtender Kunststoffschaum aus Polyvinylalkohol (PVA), der durch Anfeuchten aufweicht und seine elastischen Eigenschaften erhält.

Die WO 93 24176 A1 und die EP 0 411 632 A1 zeigen Tampons, deren Materialeigenschaften im feuchten Zustand nicht näher beschrieben werden und die zur Anwendung bei Elektrostimulationstherapien mit Oberflächenelektroden versehen sind.

Aufgabe der Erfindung ist es, einen Tampon zu schaffen, der neben der wirksamen Inkontinenz-Vorsorge bei therapeutischen Verfahren vorteilhaft einsetzbar ist.

Diese Aufgabe wird erfindungsgemäß dadurch gelöst, daß in das Material des Tampons eine unisolierte, elektrisch leitende Elektrode eingeschlossen ist, welche mit einem isolierten, aus dem Material des Tampons heraustretenden Anschlußkabel verbunden ist.

Die in das Tamponmaterial eingelassene elektrisch leitende Elektrode ermöglicht es, den Tampon als Vaginalelektrode zur Elektrostimulation zu verwenden.

Die Elektrostimulations-Therapie des Urethralbereiches dient insbesondere der Kräftigung einer erschlafften Beckenbodenmuskulatur oder des Schließmuskels der Blase. Dabei wird eine flächige Elektrode abdominal aufgebracht und eine stabförmige Elektrode vaginal eingeführt. Die zur Zeit verwendete Vaginalelektrode besteht aus inkompressiblem Material mit elektrisch leitenden Oberflächenbereichen. Sie ist relativ aufwendig und kostenintensiv in der Herstellung und für den mehrfachen Gebrauch geeignet. Nach jedem Gebrauch ist sie gründlich zu waschen und zu desinfizieren.

Der erfindungsgemäße Tampon vermag nun die bisher verwendete harte Vaginalelektrode zu ersetzen. Dabei erfolgt der Stromtransport von der Elektrode innerhalb des angefeuchteten Materials durch Ionentransport innerhalb der Flüssigkeit. Eine ausreichende Leitfähigkeit des in die Vagina eingeschobenen Tampons während einer großen Zeitdauer ist gewährleistet, da der Tampon aufgrund des durch Körperflüssigkeiten befeuchteten Umfeldes nicht austrocknen kann.

Darüber hinaus ist der erfindungsgemäße Tampon als Rektalelektrode im Falle der Therapie von Stuhlinkontinenz mittels Elektrostimulation einsetzbar. Wie oben beschrieben, entfällt gegenüber den bekannten mehrfach verwendbaren Rektalelektroden mit leitenden Oberflächenabschnitten die Notwendigkeit der Reinigung und Desinfektion.

Der Ionentransport innerhalb des angefeuchteten Tampons kann selbstverständlich in beiden Richtungen erfolgen, so daß der Tampon auch als Meßelektrode, beispielsweise zur Erstellung eines Elektromyogramms (EMG), verwendet werden kann. Bei einem bevorzugten EMG-Meßverfahren wird die Potentialdifferenz der Muskelaktivität über zwei Elektroden differentiell erfaßt und zu einer weiteren Elektrode (Masseelektrode) in Bezug gesetzt. Alle drei Elektroden können innerhalb eines erfindungsgemäßen Tampons angeordnet sein.

Schließlich können Tampons gemäß der Erfindung auch für die Iontophorese eingesetzt werden. Bei der Iontophorese werden ionisierte Medikamente mittels Gleichstrom in das menschliche Gewebe eindiffundiert. Der Tampon kann mit einer ein derartiges Medikament enthaltenden Lösung getränkt und vaginal oder rektal eingeführt werden, wobei die Gegenelektrode in der Nähe des Tampons als Oberflächenelektrode auf der Haut angebracht wird. Für die Iontophorese sind aber auch andere Einsatzorte des Tampons denkbar, z.B. in der Nasenhöhle beim Einbringen von Medikamenten über die Nasenschleimhaut. Bei dieser Anwendung des erfindungsgemäßen Tampons, bei dem die Größe der Rückstellkraft des elastischen Materials unerheblich ist, kann ein beliebiges Material, z.B. Naturschwamm oder komprimierte Watte zur Herstellung des Tampons verwendet werden.

Als Elektrode kann ein einfacher, mit einer Isolierung versehener Draht verwendet werden, dessen Ende von der Isolierung befreit und in das Material des Tampons eingeschoben ist. Der Tampon ist im Vergleich zu den bekannten Vaginalelektroden äußerst kostengünstig in der Herstellung und kann für den Einmalgebrauch vorgesehen werden. Auf diese Weise entfällt die aufwendige Desinfektion der Vaginalelektrode vor jedem Gebrauch.

Vorzugsweise weist die Elektrode ein Halteelement auf, welches von dem Material des Tampons formschlüssig umgeben ist. Beispielsweise kann die Spitze eines Drahtes auf ein scheibenförmiges oder kugelförmiges Halteelement aus elektrisch leitendem Material aufgeschweißt oder aufgelötet werden, wobei die Elektrode mit dem Halteelement bei der Herstellung des Tampons mit dem schaumartigen Material umspritzt wird.

Alternativ kann die Elektrode nach der Herstellung des Tampons in das Material eingebracht werden. In diesem Fall können die Halteelemente als Widerhaken ausgebildet sein, welche sich von der Spitze der Elektrode schräg nach hinten erstrecken. Die Widerhaken können durch an die Spitze der Elektrode angeschweißte oder angelötete Drahtstücke gebildet werden.

An dem Ende des Anschlußkabels des Tampons sollte eine elektrische Steckverbindung angeordnet sein, welche entweder direkt oder über ein Verlängerungskabel mit einem Pol des Stromausgangs eines Elektrostimulationsgerätes verbunden werden kann.

Wenn das Halteelement das Anschlußkabel ausreichend fest in dem Material des Tampons hält, kann das Anschlußkabel zum Ausziehen des Tampons aus der Vagina verwendet werden. Ist der Halt des Anschlußkabels in dem Tampon nicht ausreichend fest, kann eine separate Ausziehschnur in dem Material des Tampons verankert werden.

Schließlich betrifft die Erfindung eine Vorrichtung zur Therapie der Inkontinenz, welche neben dem erfindungsgemäßen Tampon ein Gerät zur Erzeugung des Stimulationsstroms sowie eine flächige Elektrode umfaßt, welche über ein Elektrodenkabel mit dem einen Pol des Stromausgangs des Elektrostimulationsgerätes verbunden ist, wobei der erfindungsgemäße Tampon über das Anschlußkabel mit dem zweiten Pol des Stromausgangs des Elektrostimulationsgerätes verbunden ist.

Weitere Vorzüge der Erfindung ergeben sich aus der folgenden Zeichnungsbeschreibung. Die Zeichnungen zeigen in
- Fig. 1: die schematische Darstellung eines erfindungsgemäßen Tampons, der in eine Vagina eingeführt und dessen Anschlußkabel mit einem Elektrostimulationsgerät verbunden ist,
- Fig. 2: eine vergrößerte Darstellung des Tampons aus Fig. 1 im Längsschnitt,
- Fig. 3: eine Rückansicht des Tampons aus Fig. 2,
- Fig. 4: und Fig. 5 zwei alternative Ausführungsformen des erfindungsgemäßen Tampons im Längsschnitt,
- Fig. 6: die Rückansicht des Tampons aus Fig. 5,
- Fig. 7: eine Seitenansicht eines Tampons zur Durchführung einer EMG-Messung im Längsschnitt,
- Fig. 8: die Vorderansicht des Tampons aus Fig. 7 in teilgeschnittener Darstellung und
- Fig. 9: die schematische Darstellung der an einen Meßverstärker angeschlossenen Elektroden des Tampons aus den Fig. 7 und 8.

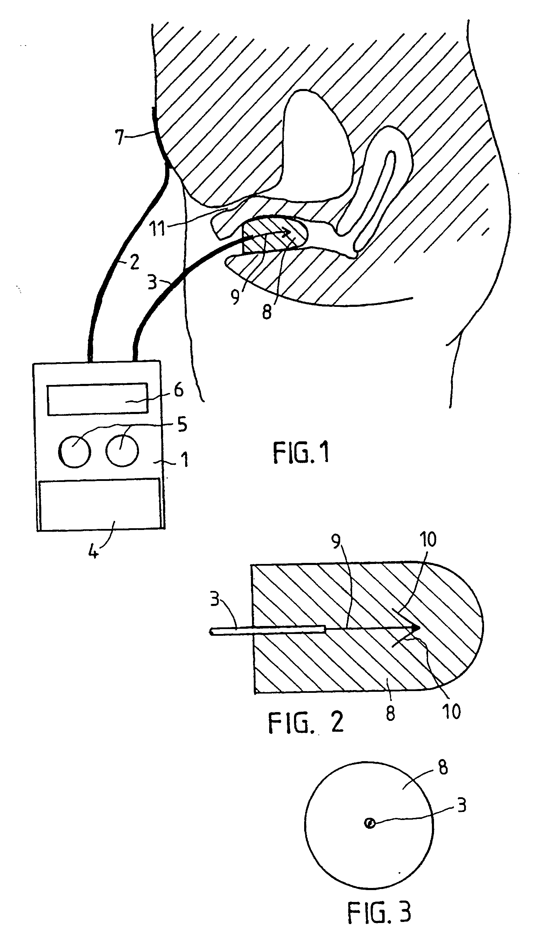

In der Fig. 1 ist eine vollständige Vorrichtung zur Therapie der Harn-Inkontinenz von Frauen mittels Elektrostimulation dargestellt. Ein Gerät 1 zur Erzeugung des Stimulationsstroms, wie es beispielsweise von der Anmelderin unter der Bezeichnung "Pro Seco" vertrieben wird, ist über zwei Kabel 2,3 mit zwei Elektroden verbunden. Das Elektrostimulationsgerät 1 umfaßt ein Aufnahmefach 4 für eine Batterie, zwei Drehregler 5 zum Regeln des Stimulationsstroms sowie eine digitale Anzeige 6. In der Praxis wird das Elektrostimulationsgerät 1 beispielsweise mittels eines Gürtels am Körper des Patienten befestigt. Eine flächige Elektrode 7, die herkömmlicherweise aus leitfähigem Kunststoff, insbesondere Silikon, besteht, wird auf der Bauchdecke des Patienten angebracht. Die zweite Stimulationselektrode wird durch das erfindungsgemäße Tampon 8 gebildet. In diesem Tampon 8 ist ein unisolierter Draht zur Bildung einer Elektrode 9 etwa axial eingebracht. Die drahtförmige Elektrode 9 ist im hinteren Bereich mit einem Kunststoffmantel umgeben und bildet außerhalb des Materials des Tampons 8 das Anschlußkabel 3. An das vordere Ende der Elektrode 9 sind zwei schräg nach hinten abstehende Drahtstücke zur Bildung von Widerhaken 10 angeschweißt. Auf diese Weise wird die Elektrode 9 sicher im Material des Tampons 8 verankert. Zum Entfernen des Tampons 8 aus der Vagina ist lediglich eine Zugkraft auf das Anschlußkabel 3 auszuüben.

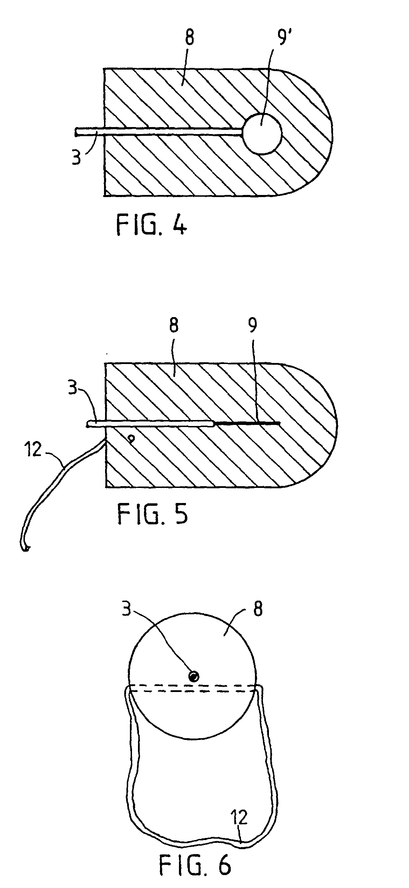

Wie in der Fig. 1 erkennbar, übt der eingeschobene Tampon 8 einen Druck auf die Urethra 11 der Trägerin aus. Hierdurch wird vermieden, daß aufgrund der Stimulation der Beckenbodenmuskulatur während der Therapie unwillkürlicher Harnverlust auftritt. In der Fig. 4 ist eine alternative Ausführungsform des erfindungsgemäßen Tampons 8 dargestellt, in der die Elektrode 9' als kugelförmiges Halteelement ausgebildet ist. Diese metallische Kugel kann nicht nachträglich in den gefertigten Tampon 8 eingeschoben werden, sondern muß mit dem schaumartigen PVA-Material bei der Herstellung des Tampons 8 umgossen werden. Die Form der als Halteelement ausgebildeten Elektrode 9' kann beliebig variiert werden und beispielsweise an den Verlauf des Stimulationsstroms oder zur Optimierung des Rückhalts des Halteelements 9' in dem Tampon 8 angepaßt werden.

Die Fig. 5 zeigt eine vereinfachte Ausführung des erfindungsgemäßen Tampons 8. Hier ist zur Bildung der Elektrode 9 einfach ein Endbereich des Anschlußkabels 3 abisoliert und in den Tampon 8 eingesteckt. Selbstverständlich hat die Elektrode 9 keinen Rückhalt in dem Tampon 8, so daß eine zusätzliche Ausziehschnur 12 durch eine Bohrung in dem Material des Tampons 8 geführt ist, mit der der Tampon 8 nach Gebrauch herausgezogen werden kann.

Die beschriebenen erfindungsgemäßen Vorrichtungen lassen sich natürlich auch zur Therapie von Stuhlinkontinenz verwenden, wobei der Tampon 8 rektal eingeführt wird und die flächige Elektrode 7 in der Nähe des Tampons 8 auf die Haut des Patienten aufgebracht wird.

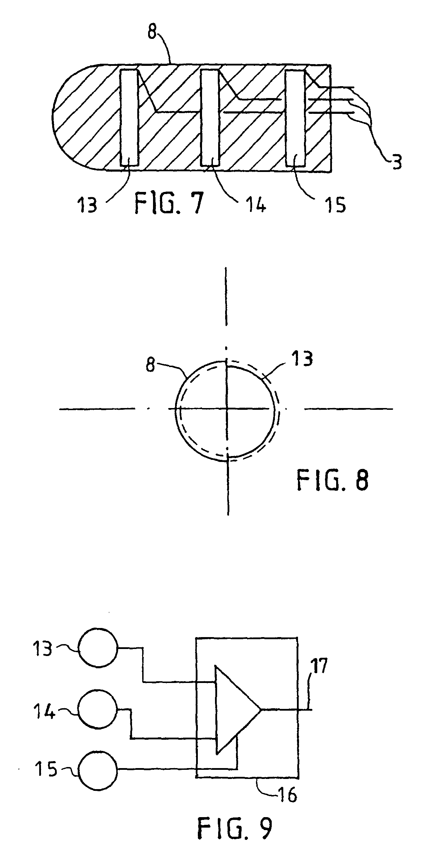

In den Fig. 7-9 ist ein Tampon zur Durchführung einer Messung zur Erstellung eines Elektromyogramms (EMG) dargestellt. Dabei ist der Tampon 8 in den Fig. 7 und 8 im Längsschnitt dargestellt, wogegen die ringförmigen Elektroden 13-15 nicht geschnitten sind.

Zwei unabhängige Elektroden 13,14 und eine Masseelektrode 15 sind innerhalb des Tampons 8 an drei unterschiedlichen axialen Positionen angeordnet. Damit die Elektroden 13-15 einen möglichst guten elektrischen Kontakt zum benachbarten Körpergewebe haben, sind sie ringförmig innerhalb des Tampons 8 mit regelmäßigen axialen Abständen zueinander angeordnet. Da die Elektroden 13-15 zur Gewährleistung eines guten elektrischen Kontaktes möglichst nahe unterhalb der Oberfläche des Tampons 8 verlaufen sollten, sind sie aus einem verformbaren, elektrisch leitenden Material, nämlich einer Metallfolie hergestellt. Die beiden Elektroden 13 und 14 sowie die Masseelektrode 15 sind an einen Meßverstärker 16 angeschlossen, der ein EMG-Meßsignal über einen Signalausgang 17 ausgibt. Von dort aus gelangt das Meßsignal zu einem geeigneten Signalaufzeichnungs- oder zu einem Signalanalysegerät.

### Bezugszeichenliste:

- 1: Elektrostimulations-Gerät
- 2: Elektrodenkabel
- 3: Anschlußkabel
- 4: Batterie-Aufnahmefach
- 5: Drehregler
- 6: Digitalanzeige
- 7: Elektrode
- 8: Tampon
- 9: drahtförmige Elektrode
- 9': kugelförmige Elektrode, Halteelement
- 10: Widerhaken, Halteelement
- 11: Urethra
- 12: Ausziehschnur
- 13: erste Elektrode
- 14: zweite Elektrode
- 15: Masseelektrode
- 16: Meßverstärker
- 17: Signalausgang

## Patentansprüche

1. Tampon (8) insbesondere zur Anwendung bei Harn-Inkontinenz von Frauen, bestehend aus einem hautverträglichen, elastischen Material, welches in angefeuchtetem Zustand entgegen einer Rückstellkraft komprimierbar ist, insbesondere aus einem schaumartigen Kunststoffmaterial wie Polyvinylalkohol (PVA), **dadurch gekennzeichnet, daß** in das Material des Tampons (8) eine unisolierte, elektrisch leitende Elektrode (9,9') eingeschlossen ist, welche mit einem isolierten, aus dem Material des Tampons (8) heraustretenden Anschlußkabel (3) verbunden ist.

2. Tampon (8) nach Anspruch 1, **dadurch gekennzeichnet, daß** das Material des Tampons eine Rückstellkraft aufweist, die bei in die Vagina eingeschobenem Tampon (8) einen Druck auf die Urethra (11) ausübt, der ausreichend groß zur Vermeidung unwillkürlichen Harnverlustes ist.

3. Tampon (8) nach Anspruch 1, **dadurch gekennzeichnet, daß** die Elektrode (9,9') ein Halteelement (9',10) umfaßt, welches von dem Material des Tampons (8) formschlüssig umgeben ist.

4. Tampon (8) nach einem der Ansprüche 1 bis 3, **dadurch gekennzeichnet, daß** die Elektrode (9,9') mit dem Kunststoffmaterial zur Bildung des Tampons (8) bei der Herstellung des Tampons (8) umspritzt ist.

5. Tampon (8) nach einem der Ansprüche 1 bis 3, **dadurch gekennzeichnet, daß** die Elektrode (9) nach der Herstellung des Tampons (8) in das Material eingebracht ist.

6. Tampon (8) nach Anspruch 5, **dadurch gekennzeichnet, daß** die Elektrode (9) stabförmig ausgebildet ist und die Halteelemente als Widerhaken (10) ausgebildet sind, welche sich von der Spitze der Elektrode (9) schräg nach hinten erstrecken.

7. Tampon (8) nach einem der vorangehenden Ansprüche, **dadurch gekennzeichnet, daß** an dem Ende des Anschlußkabels (3) ein Teil einer elektrischen Steckverbindung angeordnet ist.

8. Tampon (8) nach einem der vorangehenden Ansprüche, **dadurch gekennzeichnet, daß** eine Ausziehschnur (12) in dem Material des Tampons (8) verankert ist.

9. Tampon (8) nach einem der vorangehenden Ansprüche, **dadurch gekennzeichnet, daß** in das Material des Tampons (8) eine Mehrzahl, vorzugsweise drei, unisolierte, elektrisch leitende Elektroden (13,14,15) eingeschlossen sind, welche jeweils mit einem isolierten, aus dem Material des Tampons (8) heraustretenden Anschlußkabel (3) verbunden sind.

10. Tampon (8) nach Anspruch 9, **dadurch gekennzeichnet, daß** die drei Elektroden (13,14,15) über die Anschlußkabel (3) mit den Signaleingängen eines Meßverstärkers (16) verbunden sind.

11. Tampon (8) nach einem der vorangehenden Ansprüche, **dadurch gekennzeichnet, daß** die Elektrode (13,14,15) aus einem elektrisch leitenden und verformbaren Material, wie z.B. einer Metallfolie besteht und ringförmig innerhalb des Tampons (8) angeordnet ist.

12. Tampon (8) nach Anspruch 11, **dadurch gekennzeichnet, daß** die Elektrode (13,14,15) koaxial zur Tamponachse angeordnet ist.

13. Vorrichtung für die Inkontinenz-Therapie mit einem Gerät (1) zur Erzeugung eines Stimulationsstroms, welches einen zweipoligen Stromausgang aufweist mit einer flächigen Elektrode (7) zur abdominalen Anordnung, welche über ein Elektrodenkabel (2) mit dem ersten Pol des Stromausgangs verbunden ist, und einem Tampon (8) nach einem der vorangehenden Ansprüche zum vaginalen oder rektalen Einführen, dessen integrierte Elektrode (9,9') über das Anschlußkabel (3) mit dem zweiten Pol des Stromausgangs verbunden ist.

## Claims

1. A tampon (8) in particular for women suffering from urinary incontinence, consisting of a skin-compatible, resilient material which may be compressed counter to a restoring force when moistened, in particular of a foam-like plastics material such as polyvinyl alcohol (PVA), **characterised in that** an uninsulated, electrically conductive electrode (9, 9') is enclosed in the material of the tampon (8), which electrode is connected to an insulated connecting lead (3) extending out of the material of the tampon (8).

2. A tampon (8) according to claim 1, **characterised in that** the material of the tampon exhibits a restoring force which, when the tampon (8) has been inserted into the vagina, exerts a pressure on the urethra (11) which is sufficient to prevent involuntary urine loss.

3. A tampon (8) according to claim 1, **characterised in that** the electrode (9, 9') comprises a retaining element (9', 10) surrounded in form-fitting manner by the material of the tampon (8).

4. A tampon (8) according to any one of claims 1 to 3, **characterised in that** the electrode (9, 9') is encapsulated by injection moulding in the plastics material used to form the tampon (8) during production of the tampon (8).

5. A tampon (8) according to any one of claims 1 to 3, **characterised in that** the electrode (9) is introduced into the material after production of the tampon (8).

6. A tampon (8) according to claim 5, **characterised in that** the electrode (9) is of rod-shaped construction and the retaining elements take the form of barbs (10) extending obliquely rearwards from the tip of the electrode (9).

7. A tampon (8) according to any one of the preceding claims, **characterised in that** part of an electrical plug and socket connection is arranged at the end of the connecting lead (3).

8. A tampon (8) according to any one of the preceding claims, **characterised in that** a withdrawal cord (12) is anchored in the material of the tampon (8).

9. A tampon (8) according to any one of the preceding claims, **characterised in that** a plurality of, preferably three, uninsulated, electrically conductive electrodes (13, 14, 15) are enclosed in the material of the tampon (8), which electrodes are each connected to an insulated connecting lead (3) extending out of the material of the tampon (8).

10. A tampon (8) according to claim 9, **characterised in that** the three electrodes (13, 14, 15) are connected via the connecting leads (3) to the signal inputs of a measuring amplifier (16).

11. A tampon (8) according to any one of the preceding claims, **characterised in that** the electrode (13, 14, 15) consists of an electrically conductive, deformable material, such as a metal foil for example, and is arranged inside the tampon (8) in the manner of a ring.

12. A tampon (8) according to claim 11, **characterised in that** the electrode (13, 14, 15) is arranged coaxially relative to the tampon axis.

13. A device for treating incontinence, having an appliance (1) for generating a stimulating current, which has bipolar current output with a flat electrode (7) for abdominal arrangement, which is connected via an electrode lead (2) to the first current output pole, and a tampon (8) according to any one of the preceding claims for vaginal or rectal insertion, the integrated electrode (9, 9') of which is connected via the connecting lead (3) to the second current output pole.

## Revendications

1. Tampon (8) à utiliser notamment par des femmes souffrant d'incontinence urinaire, composé d'une matière élastique tolérée par les muqueuses et compressible, après humidification, à l'encontre d'une force de rappel, notamment d'une matière plastique de type mousse telle que l'alcool polyvinylique (PVA), **caractérisé en ce qu'**une électrode (9, 9') conductrice d'électricité et non isolée est incorporée dans la matière du tampon (8), ladite électrode étant reliée à un câble de raccordement (3) isolé qui sort de la matière du tampon (8).

2. Tampon (8) selon la revendication 1, **caractérisé en ce que** la matière du tampon a une force de rappel qui exerce, lorsque le tampon (8) est inséré dans le vagin, une pression suffisante sur l'urètre (11) pour éviter des pertes urinaires involontaires.

3. Tampon (8) selon la revendication 1, **caractérisé en ce que** l'électrode (9, 9') comporte un élément de retenue (9', 10) qui est entouré selon un engagement positif par la matière du tampon (8).

4. Tampon (8) selon l'une des revendications 1 à 3, **caractérisé en ce que** l'électrode (9, 9') est enrobée de la matière plastique destinée à former le tampon (8) au cours de la fabrication du tampon (8).

5. Tampon (8) selon l'une des revendications 1 à 3, **caractérisé en ce que** l'électrode (9) est introduite dans la matière après fabrication du tampon (8).

6. Tampon (8) selon la revendication 5, **caractérisé en ce que** l'électrode (9) est en forme de baguette et **en ce que** les éléments de retenue sont conçus sous la forme d'ardillons (10) qui s'étendent en oblique de la pointe de l'électrode (9) vers l'arrière.

7. Tampon (8) selon l'une des revendications précédentes, **caractérisé en ce qu'**un élément d'une connexion électrique enfichable est agencé à l'extrémité du câble de raccordement (3).

8. Tampon (8) selon l'une des revendications précédentes, **caractérisé en ce qu'**un cordon de retrait (12) est ancré dans la matière du tampon (8).

9. Tampon (8) selon l'une des revendications précédentes, **caractérisé en ce que** plusieurs électrodes (13, 14, 15) conductrices d'électricité et non isolées, trois de préférence, sont intégrées dans la matière du tampon (8), lesdites électrodes étant chacune reliées à un câble de raccordement (3) isolé qui sort de la matière du tampon (8).

10. Tampon (8) selon la revendication 9, **caractérisé en ce que** les trois électrodes (13, 14, 15) sont reliées aux entrées de signaux d'un amplificateur de mesure (16) par l'intermédiaire du câble de raccordement (3).

11. Tampon (8) selon l'une des revendications précédentes, **caractérisé en ce que** l'électrode (13, 14, 15) est composée d'une matière conductrice d'électricité et déformable, par exemple un film métallique, et est agencée selon une forme annulaire à l'intérieur du tampon (8).

12. Tampon (8) selon la revendication 11, **caractérisé en ce que** l'électrode (13, 14, 15) est agencée coaxialement au tampon.

13. Dispositif destiné au traitement de l'incontinence comportant un appareil (1) destiné à produire un courant de stimulation, appareil qui présente une sortie de courant bipolaire avec une électrode plane (7) à disposer sur l'abdomen, laquelle électrode est reliée via un câble d'électrode (2) au premier pôle de sortie de courant, et comportant un tampon (8) selon l'une des revendications précédentes destiné à une introduction vaginale ou rectale, dont l'électrode (9, 9') intégrée est reliée au deuxième pôle de sortie de courant via le câble de raccordement (3).
